# EUROPEAN PATENT APPLICATION

(11) **EP 4 379 733 A1**
(43) Date of publication of application: **05.06.2024**
(21) Application number: 22210675.9
(22) Date of filing: 30.11.2022
(51) Int. Cl.: G16H 20/10

(54) **VISUALIZATION OF MEDICAL INTERVENTION EFFECT**

(71) Applicant: Koninklijke Philips N.V., 5656 AG Eindhoven (NL)
(72) Inventor: HUIJBREGTS, Laurentia Johanna, 5656AG Eindhoven (NL); VAN LIESHOUT, Ron Martinus Laurentius, 5656AG Eindhoven (NL)
(74) Representative: Philips Intellectual Property & Standards

(57) **Abstract**

Some embodiments are directed to a visualization device for showing a patient status. The visualization device may compute an effect that a medical intervention is expected to have on the patient and generate a visualization representing a physiological parameter and the expected effect. The generated visualization may be displayed on a display.

## Description

### FIELD OF THE INVENTION

The presently disclosed subject matter relates to a visualization device, a visualization method, and a computer readable medium.

### BACKGROUND OF THE INVENTION

Visualizations have been developed where, from a drawing, in one view, the patient status can be seen. For example, a visualization may show advanced hemodynamic measurements to support clinical decision-making.

On the ICU, the patient's vital signs (heart rate (HR), electrocardiography (ECG), respiration rate (RR), core body temperature (CBT), oxygen saturation (SpO2) and blood pressure (BP)) are closely monitored. However, sometimes it is not enough to monitor only the regular vital signs, and advanced hemodynamic measurements are needed to keep a better eye on the patient with hemodynamic complications and/or to diagnose the cause of these complications. Among hemodynamic parameters to be measured are cardiac output (CO), stroke volume (SV), stroke volume variation (SVV), ejection fraction (EF), cardiac index (CI), systemic vascular resistance (SVR), pulmonary vascular resistance (PVR), pulmonary artery pressure (PAP), and extravascular lung water index (ELWI). There are various modalities available to measure hemodynamic parameters, varying in which parameters they measure, their invasiveness, and their accuracy. Examples are a Swan Ganz pulmonary artery catheter (PAC), PiCCO (which combines an arterial line and a central venous line), Edwards ClearSight (which uses finger cuffs), Edwards FloTrac (using an arterial line), and echography.

### SUMMARY OF THE INVENTION

Current visualizations only show the patient's physiological parameters and not the interventions that the patient underwent. Adding the interventions to the view enables analyzing the patient's response to these interventions.

Furthermore, these visualizations do not show the effects of any interventions that the patient underwent.

It would be advantageous to show the effect, e.g., therapeutic effect of an intervention, e.g., an applied medication, e.g., going towards the peak plasma concentration and/or wearing off, or of a fluid challenge reaching peak effect. Such effect may be shown together with physiological parameters indicative of a patient status. Such a display assists a medical practitioner in making clinical decisions. For example, although the effect of a medication is at peak, the shown physiological parameters may not show sufficient change. At that point a practitioner may consider increasing or changing medication. Likewise, a practitioner may notice a discrepancy between a nominally expected effect level, and the actual physiological parameters, which may give the practitioner further insight in the status of the patient.

For example, the visualization device obtains physiological parameters indicative of a patient status and information regarding a medical intervention that has been applied to the patient at a first point in time. The effect of the medical intervention, e.g., of an administered substance, such as medication or a fluid, e.g., a fluid challenge, may be shown at one or more second time points after the first time point. For example, such an expected effect may be computed, e.g., using pharmacological models, possibly assisted by relevant information, such as weight, or sex of the patient and possibly measured values.

A visualization may be generated that represents one or more physiological parameters and expected effect in a single visualization. The visualization may be shown on a display. In an embodiment, the physiological parameters may be shown in real time, and expected effect may correspond to the current moment.

Visualization may show a current intervention effect and/or physiological parameter, e.g., corresponding to the moment in time the visualization is shown. In an embodiment, a visualization may show historic intervention effect and/or physiological parameter, e.g., corresponding to a moment in the past. A visualization device may comprise an input interface, e.g., a slider, a knob, etc., configured to receive a time point.

The input interface may be configured to allow sliding through time. Instead of manual sliding, e.g., by a user such as a caregiver, a visualization system may show the time elapse with video animation. The video could show chronologically, e.g., from past to present, or reverse chronological, e.g., starting at the current time and moving backwards in time. The animation may use a constant speed, e.g., every minute of data takes 1 second in the video, or the animation speed may change. For example, the animation speed may adapt to the medication effect, e.g., when the medication effect is large, the video plays slowly, or to the physiological parameters, e.g., when one or more physiological parameters change a lot and/or fast, the video plays slowly, whereas it plays fast for the parts where changes are small.

A medication effect is an example of an effect of a medical intervention. Other interventions than medication include interventions such as applied fluids or ventilation. An embodiment showing medication effect may be adapted to an embodiment showing a ventilation effect, fluid effect, or more general effect of a medical intervention.

The visualization device is an electronic device; the visualization device may comprise a computer.

A further aspect is a visualization method. An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

Another aspect of the presently disclosed subject matter is a method of making the computer program available for downloading.

### BRIEF DESCRIPTION OF DRAWINGS

Further details, aspects, and embodiments will be described, by way of example, with reference to the drawings. Elements in the figures are illustrated for simplicity and clarity and have not necessarily been drawn to scale. In the Figures, elements which correspond to elements already described may have the same reference numerals. In the drawings,
Fig. 1 schematically shows an example of an embodiment of a visualization device,
Fig. 2a schematically shows an example of an embodiment of a visualization,
Fig. 2b schematically shows an example of an embodiment of a visualization,
Fig. 2c schematically shows an example of an embodiment of a visualization,
Fig. 2d schematically shows an example of an embodiment of a visualization,
Fig. 3 schematically shows an example of an embodiment of a visualization,
Fig. 4a schematically shows examples of an embodiment of a visualization representing an expected effect at a particular point in time,
Fig. 4b schematically shows examples of an embodiment of a visualization representing an expected effect at a particular point in time,
Fig. 4c schematically shows examples of an embodiment of a visualization representing an expected effect at a particular point in time,
Fig. 4d schematically shows examples of an embodiment of a visualization representing an expected effect at a particular point in time,
Fig. 5 schematically shows an example of an embodiment of a visualization method,
Fig. 6a schematically shows a computer readable medium having a writable part comprising a computer program according to an embodiment,
Fig. 6b schematically shows a representation of a processor system according to an embodiment.

### Reference signs list

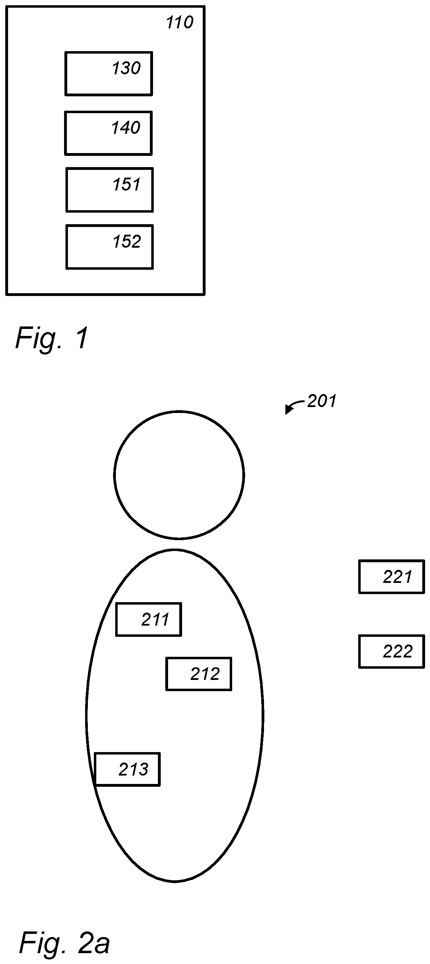

The following list of reference signs refers to figures 1, 2a-2d, 3, 6a and 6b, and is provided for facilitating the interpretation of the drawings and shall not be construed as limiting the claims.
- 110: a visualization device
- 130: a processor system
- 140: storage
- 151: communication interface
- 152: display interface

- 201: a graphical model of at least part of the patient indicating one or more physiological parameters
- 211-213: an indicated physiological parameter
- 221, 222: a visualized expected effect at a time point
- 231, 232: a graph of a physiological parameter
- 233: a timeline
- 241, 242: a medical intervention
- 251, 252: a visualized expected effect at a time point
- 261, 262: a visualization of physiological parameter trend against time
- 271: a plot of two physiological parameters against each other
- 272: a medical intervention
- 273: a time point
- 301: normal ventricular systolic function
- 305: poor ventricular systolic function
- 310: cardia preload axis
- 311: fluid challenge
- 320: stroke volume axis
- 321: no response
- 322: significant response
- 331: point of plot
- 332: plot of stroke volume versus cardiac preload
- 351: effect visualization

- 1000: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only, but also includes every other combination of features described herein or recited in mutually different dependent claims.

Fig. 1 schematically shows an example of an embodiment of a visualization device 110.

Visualization device 110 is configured to show a visualization of a patient status. For example, the visualization device 110 may show a visualization of physiological parameters indicative of a patient status. Visualization device 110 also shows a visualization of an expected effect of a medical intervention applied to a patient earlier. For example, the effect of a substance applied to the patient, e.g., a medication or a fluid, is not constant. Visualization device 110 allows a medical practitioner to quickly learn the effect that a previous medical intervention has at a later time point, in particular at the current time point.

For example, the device 110 may be used in a clinical setting, e.g., to monitor a patient. Medical personnel can view the visualization and know quickly, not just the physiological parameters, but also the effect, e.g., the remaining effect of previous medical interventions. Knowledge of such remaining effect is important, e.g., when deciding on new interventions, evaluation the previous interventions, and the like. A medical professional with knowledge in this area is capable of estimating such effects. However, the device offers several advantages. It is less error-prone and quicker. Moreover, it offers the information also to medical professional without the required training or experience to estimate effects.

In an embodiment, visualization device 110 is configured for monitoring a patient. For example, visualization device 110 may be configured for bedside placement.

For example, visualization device 110 may be configured for placement at the central station in the hospital. Alternatively or additionally, a display may be placed at the bedside, configured to display a visualization generated by device 110. Computations, e.g., effect and visualization computations may be split over two locations as well. Visualization device 110 may be a mobile device, e.g., a mobile phone, such as the mobile phone of a caregiver.

For example, visualization device 110 may be configured for evaluating patient data, possibly historic patient data. For example, a workstation or imaging apparatus may comprise visualization device 110.

An embodiment may be used in combination with an electronic medical records (EMRs). For example, in an embodiment a device may be configured for searching and/or showing electronic medical record. A record may comprise an intervention applied to a patient. The device may be configured to determine and/or visualize the effect over time that follows from the intervention.

An embodiment may be applied to patient monitoring, e.g., in an ICU. However, it can also be used for patient monitoring in lower acuity settings in the hospital or for patients at home, where a diagnosis and/or treatment plan is needed. Instead of being directly used on the patient monitor, it could be used in an EMR or any other health record. In one embodiment, the user himself choses the times to show. In another embodiment, the system selects the most interesting time points and shows those. In an embodiment, the caregiver can scroll through time.

Visualization device 110 may comprise a processor system 130, a storage 140, a communication interface 151, and a display interface 152. Storage 140 may be, e.g., electronic storage, magnetic storage, etc. The storage may comprise local storage, e.g., a local hard drive or electronic memory. Storage 140 may comprise non-local storage, e.g., cloud storage. In the latter case, storage 140 may comprise a storage interface to the non-local storage. Storage may comprise multiple discrete sub-storages together making up storage 140. Storage may comprise a volatile writable part, say a RAM, a non-volatile writable part, e.g., Flash, a non-volatile non-writable part, e.g., ROM. Device 110 may be implemented in a distributed fashion; for example, effect computations may be performed on a processor at a geographically different location than, say, the processor computing visualizations. Device 110 may be configured local computation, e.g., performing all computations locally, even if storage may or may not be local.

Storage 140 may be non-transitory storage. For example, storage 140 may store data in the presence of power such as a volatile memory device, e.g., a Random Access Memory (RAM). For example, storage 140 may store data in the presence of power as well as outside the presence of power such as a non-volatile memory device, e.g., Flash memory.

The device 110 may communicate internally, with other systems, with other devices, external storage, input devices, output devices, and/or one or more sensors over a computer network. The computer network may be an internet, an intranet, a LAN, a WLAN, etc. The computer network may be the Internet. The device 110 comprise a connection interface which is arranged to communicate within system 100 or outside of system 100 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

The communication interface 151 may be used to receive digital data, e.g., physiological parameters. The communication interface 151 may be used to send digital data, e.g., effect data, visualization data, etc. Communication interface 151 may comprise an input interface and/or an output interface.

Display interface 152 may be configured to enable a display to show a visualization of the patient status. For example, the display interface may comprise an HDMI interface, a display port interface, or the like. For example, the display interface may be configured to transfer display signals configured to control a display. For example, the display interface may comprise a computer network interface. The display interface may comprise an external interface, e.g., configured to connect wirelessly or wired to an external display, e.g., a monitor. The display interface may comprise an internal interface, e.g., configured to an internal display, e.g., a monitor. The display may be an LCD monitor, an OLED monitor, etc.

The execution of device 110 may be implemented in a processor system. The device 110 may comprise functional units to implement aspects of embodiments. The functional units may be part of the processor system. For example, functional units shown herein may be wholly or partially implemented in computer instructions that are stored in a storage of the device and executable by the processor system.

The processor system may comprise one or more processor circuits, e.g., microprocessors, CPUs, GPUs, etc. Device 110 may comprise multiple processors. A processor circuit may be implemented in a distributed fashion, e.g., as multiple sub-processor circuits. For example, device 110 may use cloud computing.

Typically, the visualization device 110 comprises a microprocessor which executes appropriate software stored at the device; for example, that software may have been downloaded and/or stored in a corresponding memory, e.g., a volatile memory such as RAM or a non-volatile memory such as Flash.

Instead of using software to implement a function, device 110 may, in whole or in part, be implemented in programmable logic, e.g., as field-programmable gate array (FPGA). The device may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, visualization device 110 may comprise circuits, e.g., for arithmetic processing.

In hybrid embodiments, functional units are implemented partially in hardware, e.g., as coprocessors, e.g., neural network coprocessors, and partially in software stored and executed on the device.

A visualization device helps clinicians interpreting the physiological parameters of the patient and their response to interventions. The visualization device may comprise a display, a display interface configured to connect to a display. The display may be configured as a patient monitor, EMR, or a separate display, etc. For example, a page on the display may show past and/or present physiological signals of a patient, an image indicating a type of intervention on the patient and its magnitude and/or trend, e.g., its effect at a later time point.

Various interventions may be visualized. In an embodiment, the intervention may comprise the administering of medication, fluid therapy and/or a fluid challenge. The time at which the intervention was administered may be visualized. Furthermore, or instead, the magnitude and/or trend of its effect may be visualized. For example, it may give an indication of the time for intervention effect to increase and/or decrease. For example, a period between the therapy starting to work, e.g., becoming operative, e.g., having a first noticeable concentration and/or therapeutic effect, and later on the fading out. For medication this may indicate the magnitude and/or trend of the concentration of the medication in blood.

### Physiological parameters

The visualization device may be configured to receive physiological parameters indicative of a patient status, e.g., through a communication interface. For example, sensors applied near, on, or in a patient may register physiological parameters of the patient. For example, a sensor may be configured to send sensor values to the visualization device, e.g., over a computer network or the like.

For example, in an embodiment at least one, at least two, at least four, physiological parameters are received and visualized. Many physiological parameters may be used, e.g., such as is known in conventional visualization devices.

For example, the physiological parameters comprise:
- one or more vital signs, including one or more of: heart rate (HR), electrocardiography (ECG), respiration rate (RR), core body temperature (CBT), oxygen saturation (SpO2) and blood pressure (BP), and/or
- one or more hemodynamic parameters from advanced hemodynamic measurements, including one or more of: cardiac output (CO), stroke volume (SV), stroke volume variation (SVV), ejection fraction (EF), cardiac index (CI), systemic vascular resistance (SVR), pulmonary vascular resistance (PVR), pulmonary artery pressure (PAP), and extravascular lung water index (ELWI),
- one or more of blood parameters, including blood gas, e.g., blood pH, blood COOH, and/or blood values, e.g., blood Hb, blood Ht, blood Hct, and/or
- urine output.

For example, such parameters may be obtained by a corresponding sensor appropriately configured for measuring the parameter. An embodiment may be restricted to vital signs that are relatively easy to measure, or may also or instead use more advanced hemodynamic monitoring. Hemodynamic monitoring may use a heart sensor, e.g., comprising a central venous pressure (CVP) catheter and/or thermodilution arterial line.

Besides the parameters that were already mentioned above, other parameters from advanced hemodynamic measurements exist, for which the invention may also apply. An example of a further parameter is cardiac output, e.g., the global ejection fraction (GEF) e.g., measured by PiCCO, or ejection fraction (EF), which may be measured using an ultrasound sensor. Either EF or GEF may be visualized, e.g., in a patient model or the like, depending on which measurement modality is used. Two further parameters that we may be visualized are the pulmonary artery wedge pressure (PAWP) and global end-diastolic volume index (GEDVI), (or its non-indexed version GEDV). Note that for some parameters indexed values are sometimes used, which take into account the patient's body surface area. Those parameters may be indicated with an 'I' at the end; SVI, ELWI, CI, PVRI, SVRI, GEDVI, instead of respectively SV, EVLWI, CO, PVR, SVR, GEDV, etc. A patient model may show indexed or non-indexed parameters. Cardiac output or its indexed version cardiac index, may be measured continuously instead of intermittently. For the continuous cardiac output and continuous cardiac index the acronym CCO and CCI are used, whereas for the intermittent measurements the acronyms CO and CI are used.

The particular list of all parameters that may be shown in a visualization is long and varied, and will depend on the purpose of a particular embodiment. For example, for cardiac monitoring in a recovery facility other parameters may be visualized than during active surgery.

### Patient status visualization

The visualization device may comprise a display interface configured to enable a display to show a visualization of the patient status. The visualization device may be configured to generate a visualization representing the physiological parameter at the one or multiple time points. There are various options to choose the time points for which physiological parameter are visualized.

For example, the physiological parameter visualization may visualize the physiological parameter of a single time point-typically the current time point. For example, current patient status may be shown in real time. For example, clinical personnel could navigate through time, while the visualization corresponds to a timepoint selected by the personnel.

For example, the physiological parameter visualization may visualize the physiological parameter at multiple time points-typically the current time point in addition to a series of recent time points leading up to the current time point. For example, current patient status may be shown in real time. The multiple time points may include a first time point at which a medical intervention was applied and a second time point at which the effect of the medical intervention is shown. Showing the first time point at which a medical intervention was applied is optional.

For example, a sequence of physiological parameter visualization for a sequence of time point, e.g., forming a video showing development of the physiological parameter.

Various types of visualizations are possible; in particular conventional patient status displays could be modified according to an embodiment. Various examples of visualization are discussed below.

### Medical interventions

At some point in time, a medical intervention has been applied to the patient. A typical intervention is the administration of a substance, e.g., a pharmacological active composition, e.g., a medicine, e.g., a pill, e.g., a fluid. The substance could be taken orally or administered through an IV, etc. For example, an embodiment could be usefully applied to the administration of noradrenaline.

The visualization device may obtain information indicating the medical intervention that has been applied to the patient at a first point in time in various ways. For example, the information may be directly entered at the visual device, e.g., through a keyboard, touchscreen, or the like. The interventions may be obtained from another device. For example, the interventions may be obtained from an electronic medical record (EMR). For example, the interventions may be obtained from an electronic medical device, e.g., an infusion pump, or a ventilator. For example, such information may be received over an electronic connection, e.g., a computer network. The visualization device may be integrated in the other device. For example, the visualization device could be part of an EMR server.

Different types of interventions include further: medication that is injected, administered via IV-pump, oral medication in liquid form, oral medication as pills, fluid bag, oxygen therapy, mechanical ventilation, milk for neonate. Information regarding the type of intervention could be included in the visualization, including effect visualization, as further discussed herein.

### Compute an expected effect of medical intervention

The visualization device may be configured to compute an effect that the medical intervention is expected to have on the patient at a second point in time. That is, at some point in time after the intervention was administered. The visualization device may be configured to compute such an effect at multiple second points in time.

In an embodiment, the visualization device is configured to compute the effect for the current time point, e.g., to allow a real time view of the current effect of the intervention, e.g., of some medication that was applied. The visualization device could compute the effect for multiple time points, so that the user could navigate through time, e.g., by sliding on a timeline that shows the physiological parameters over time, and see the effect at each time point that he selects. The effect may be graphed, e.g., against one or more physiological parameters.

In an embodiment, an image is shown at a selected time. The image may show a patient model, etc. For example, the image may show trends of parameters on one side of the image. A graphical user interface element, e.g., a slider, may be shown in the image configured to allow a user to input time. Using the GUI element, e.g., dragging the slider, the image, e.g., may show a parameter and a medication effect at the time indicated by the GUI element. In an example, the image shows only parameter trends and medication effect at the selected time.

For example, the intervention may comprise administration of a medically active composition, in which case the effect may comprise a computed concentration in the patient, such as a plasma concentration. Note that there may be multiple medical interventions, the effects of which overlap in time. Such cases are especially hard to judge for practitioners without computed help.

Computing the expected effect for a given second time point, given an intervention at a first time point, or first time points, may comprise applying a pharmacokinetic model. The pharmacokinetic model may model quantitatively a drug movement in, through and out of the body.

For example, a first pharmacokinetic model may model the absorption and excretion of a drug. For example, a second pharmacokinetic model may model the absorption of a drug given its dosage and dosage form, the distribution and storage of the drug, both in blood and outside, e.g., in various storage tissues and/or at the site of action, the binding and unbinding in tissue, and the excretion of the drug, e.g., through urine, bile, feces, sweat and salvia. The pharmacokinetic may also take into account the biotransformations that are expected to take place, e.g., between protein, metabolites, etc. The pharmacokinetic model may take as input the medical intervention, but also other patient parameters, e.g., patient weight, the first time point, etc.

The model may be simplified if needed. For example, a third model may comprise a decay function taking as input the intervention, e.g., a dosage, and possibly one or more patient parameters, and approximate therefrom the effect. For example, such a function may be an exponentially decaying function.

For example, the system may obtain the time of administration, e.g., taken from manual input or from the infusion pump data, etc., and calculate the further trend by using typical trends, such as half-life time, for the type of medication, based on literature or previous measurements on other patients. Such calculations might take into account the height, weight, and/or BMI of the patient. Such calculations might take into account parameters measured of the patient, e.g., values in sweat, urine, exhaled breath, or the like, e.g., correlated to medicine or fluid excretion. See the paper "Online Real-Time Monitoring of Exhaled Breath Particles Reveals Unnoticed Transport of Nonvolatile Drugs from Blood to Breath", by Xing Chen, et al., (incorporated by reference) for more information on monitoring exhaled breath.

The typical times from literature and/or from previous measurements on patients might be pre-programmed into an embodiment or might get updated/retrieved regularly. When connected with an infusion pump, they might also be retrieved from the medication library of the infusion pump.

Alternatively, shown trends might be based on actual blood concentration measurements of the blood of the patient. Alternatively, biomarkers in the patient's sweat are used, preferably taking into account the delay time for the substance to transfer from blood to sweat. Such blood or sweat measurements are of particular interest when the pharmacokinetics (in particular typical time until becoming operative and having a therapeutic effect and typical half-life time) of the particular drug is not yet known (e.g., from literature) and/or if there is a large spread in typical start-up time and/or half-life times between different patients for the particular drug.

Due to these biological processes, some or all or none of which may be modelled, the majority of medications take some time to act. Especially for oral intake, it may take a considerable time before the active substance reaches the blood. For example, for seizure medicines, it takes between 30 min and 6 hours before maximum concentration in blood has been reached, depending on the exact type of medicine. For medication administered directly to the blood, this time is considerably lower, but still some time is needed before the substance has been distributed in the blood throughout the body. After the maximum concentration has been reached, the concentration will start declining, typically with a half-life time, which also depends on the type of medication. The medication then gets excreted from the body via urine, feces or sweat.

The effect computation may use stored trendlines of effect, e.g., of blood concentrations, which are known, e.g., previously measured. Such stored trendlines may be stored at the visualization device, e.g., in the form of a look-up table.

Note that the above explained pharmacokinetics for administration of discrete portions of medication. Instead, medication can be administered continuously via an infusion pump. After an initiation phase, the concentration in the blood is expected to become constant over time if the administered concentration is constant. This may be taken into account as well, if desired. For example, the point of application could be taken as the start or end of the infusion pump.

Typically, it will be assumed that the moment in time of the first time point, e.g., of the medical intervention is known with sufficient accuracy. However, it may happen that this is not known with enough accuracy. For example, one of the problems with an EMR system is that the time-stamp linked to the intervention is generated either by the entry within the EMR or by a manual annotation, which may not always be accurate. In an embodiment, the accuracy of the first time point may be improved by estimating it from a measured effect. For example, this may use the same pharmacological model as above, but applied in reverse, to calculate back the moment of intervention from observed effects. Once the time point of the intervention has been estimated, its further effects, e.g., later effects, can be predicted. For example, the first time point is estimated from a measured effect of the intervention, and the expected effect at the second time point.

### Visualize expected effect

The visualization device is configured to generate a visualization representing the physiological parameter at the first and/or second time point, and the expected effect at the second time point. The visualization device may be configured for causing the generated visualization to be displayed on the display through the display interface.

For example, the visualization may represent the physiological parameter at the second time point, and the expected effect at the second time point.

For example, the visualization may represent the physiological parameter at the first and second time point, and the expected effect at the second time point.

For example, the visualization may represent the physiological parameter at multiple times points between the first and second time point, and the expected effect at the second time point.

For example, the visualization may show the physiological parameter and the expected effect in real time. For example, the second time point may be the current time point.

The visualization of the effect may be shown on a patient monitor where vital signs are also displayed in a live manner. For example, the current measurements of the vital signs may be shown. For example, in an embodiment, a patient monitor may show-together with current values of the patient's vital signs-which medication is currently having influence on the patient. For example, a first medication have a declining effect, e.g., a declining concentration in blood, whereas a second medication is being administered at a rate which has recently been lowered and there is still an effect of the higher rate it had before.

An embodiment visualizes the medication becoming effective in after administration and the medication fading out after the maximum effect has been reached. This can be done by using an icon representing a certain type of medication which changes in time, e.g., because of increasing and decreasing blood concentration. The icon change may comprise transparency, darkness/brightness, color, and/or size, or an additional arrow may represent the slope of the medication concentration in the blood.

In an embodiment, the effect of the intervention is visualized for at least one time point-the second time point. The medical intervention itself, e.g., visualizing the moment in time the intervention happened is possible, but not necessary. For example, there could be a momentary view of the physiological parameters at the second time point, without showing the physiological parameters for any other time point. For example, data could be shown for a timeframe around the second time point but not including the first time point. In those cases, the medical intervention at the first time point, and even the physiological parameters at the first time point, would not show up as visualization. Typically, the second time point corresponds to the moment when the generated visualization is displayed, but this is not necessary; for example, the second time point could be in the past. In another example, the physiological parameters at the first time point are included in the visualization, but the medical effect is only shown for a certain selected time point, e.g., the second time point. For example, trendlines of parameters and the medication effect may be shown only for a time point or interval indicated by a user, e.g., through a slider. For example, two or more parameters may be plotted against each other, e.g., in a Frank-Starling curve, in which case, the image may be for a specific time or time interval.

Various classes of example visualization are discussed and/or illustrated; discussed are examples A, B, C and D.

### A. Snapshot of current physiological parameter and effect

An example of an embodiment of such a visualization is schematically shown in Fig. 2a. For example, in an embodiment, the generated visualization may represent a snapshot of the physiological parameter at the second time point. This is an example of patient status visualization. The effect of the medical intervention may be a current effect, e.g., an expected effect at the moment of visualization, also referred to as a momentary effect. An expected effect may also be an effect in the past. Visualization of an effect may advantageously include a trend indication of the effect, e.g., indicating if the effect is expected to increase of decrease. Showing a trend is not necessary.

For example, the visualization representing the physiological parameter may comprise a graphical model of at least part of the patient indicating one or more physiological parameters, such as a cardiopulmonary (CP) model or visual representation of the patient.

Fig. 2a shows a graphical model of at least part of the patient indicating one or more physiological parameters. In this case, a schematic drawing of the upper part of a human body is shown. In or near the graphical model one or more physiological parameters are indicated. Shown are three parameters: 211-213. For example, the indicated physiological parameter may be one or more of the physiological parameters discussed herein. The indicated parameters are preferably indicated visually, for example, an increased blood pressure may be shown by a changed icon, e.g., having an increased size, appearance, color and so on. Examples of such graphical models of at least part of a patient are the visual patient described in the paper "Avatar-based patient monitoring in critical anesthesia events: a randomized high-fidelity simulation study", by T.R. Roche et al. (included herein by reference) and the figure-eight schematic that represents a circulatory system of the patient, described in co-pending application EP4014851A1 of the applicant with title 'Graphical representation of hemodynamic state' (included herein by reference).

Fig. 2a also shows two visualized expected effects at a time point: 221 and 222. For example, these may be icons indicating the remaining effect of two previous interventions. More or fewer effects than 2 may be shown.

Although some clinical professionals, e.g., intensivists, are capable of interpreting the values of the advanced hemodynamic measurements, usually physician assistants, residents, and nurses are less familiar with them. To help them interpret the values, a model may be used to visualize the values. For example, this may be a model such shown in Fig. 2a.

For example, the visual model may be a cardiopulmonary (CP) model. The CP model may show, e.g., a picture with an upper loop that represents the pulmonary circulation, a lower loop that represents the systemic circulation, and a central object that represents the functional parameters of the heart. Physiological measurements may then be displayed in various ways on visual model, e.g., from the thickness of part of the loops, the blood pressure or vascular resistance can be seen. Optionally lungs are drawn and their filling represents the amount of lung water. Such a CP visualization is an example of patient status visualization.

In general, patient status visualization may comprise a picture that gives a visualization of physiological parameters of a patient. The physiological parameters could be one or more of advanced hemodynamic parameters, regular vital signs, biomarker/hormones/neurochemical/electrolyte concentrations in blood, in sweat or in expired air, etc. They relate to the health of the patient and can change over time. Some may fluctuate within minutes or hours; others show only considerable changes over days or months.

Other patient status visualizations could be applicable to other organ system-based physiological visualizations (beyond hemodynamics) such as neurological, renal, respiratory.

By showing in or near the graphical representation additional icons that represent the effect or remaining effect of previous interventions, the information conveyed in the display is improved. The user will at a glance obtain information needed to evaluate the desirability of further interventions. Furthermore, it becomes much easier to evaluate the current status in view of the effect of medicine. For example, a low blood pressure would be understandable if a medicine which lowers blood pressure is still active.

Icons to indicate expected effect of interventions a physiological parameter may be shown in a similar style. For example, icon size, color, or animation, and so on, may indicate both elevated values.

A trend of a medication effect or of a parameter may be indicated, e.g., with arrows in the image, but this is not necessary. Instead, the visualization could only show the current parameter or current effect.

Showing a trend view of physiological parameters, e.g., next to a patient status visualization is not necessary. For example, an embodiment may show only the patient status visualization and medication effect, without trends over time, e.g., with physiological parameters next to it.

Instead of showing one or more of a current value of a parameter, current effect of an intervention, a current trend of the parameter, a current trend of the effect, e.g., the value, effect or trend corresponding to the time or near the time of showing the visualization, an embodiment may also or instead show historic values. For example, in an embodiment, a user may select a time in the past and the snapshot may then be visualized, showing parameters at that point in time and the medication effect at that point in time. The time point does not necessarily need to be current. For example, a visualization device may be configured to receive a time input indicating a point in time, or an interval for which the visualization is performed. In that case, one or more of a historic parameter, historic intervention effect, historic parameter trend, historic intervention effect, etc., may be shown. The time of the snapshot may be selected by the system to be a time instance which is deemed of particular relevance for the intervention. For example, the time at which the effect of the intervention is assumed to be at a maximum. The system may show multiple snapshots (either together on one screen, or after each other), each belonging to an interesting time point; e.g. first before the intervention, then when the effect of the intervention is at maximum, and finally when the effect of the intervention has declined to 10 % of its maximum.

### B. Trendline physiological parameter and effect

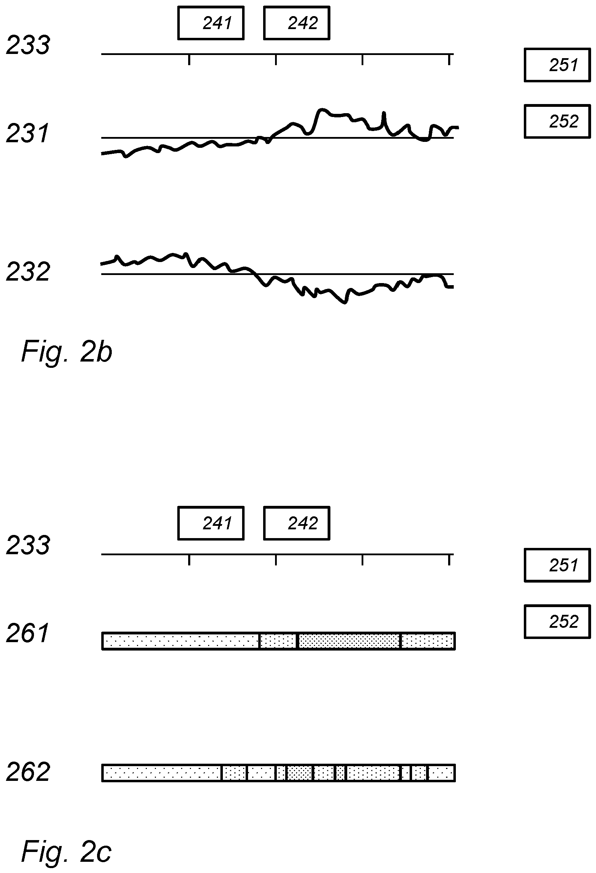

An example of an embodiment of such a visualization is schematically shown in Fig. 2b and Fig. 2c. Optionally these views may also show the medical intervention itself. For example, the visualization may represent the physiological parameter as a trend view of the physiological parameter or a derived parameter against time, wherein the parameters is plotted against time.

The effect of the medical intervention may be a current effect, e.g., an expected effect at the moment of visualization, also referred to as a momentary effect. An expected effect may also be an effect in the past. Visualization of an effect may advantageously include a trend indication of the effect, e.g., indicating if the effect is expected to increase or decrease. Showing a trend is not necessary.
To be able to see if a patient is getting better, is stable, or is deteriorating, or to see if certain interventions, e.g., medication and/or fluids, have an effect, it may be important to look at the historical trends of the measurements. In some conventional patient monitors, trend views have been integrated. An embodiment may include such a trend view as well. For example, one may show trends of the values of, say, the regular vital signs and/or more advanced hemodynamic measurements.

**Fig. 2b** and **Fig. 2c** schematically show examples of an embodiment of a visualization.

Fig. 2b shows two graphs: graph 231 and graph 232. Each graph plots a physiological parameter against time. The horizontal (time) axis may cover, e.g., 30 minutes, 4 hours, 8 hours, or 24 hours, or more, or less. More or fewer physiological parameters may be shown in the visualization. For convenience an optional timeline 233 is also included in the visualization.

Fig. 2b shows a visualization of an expected effect of a previous medical intervention. Shown are effects 251 and 252. Similar visualization of such effects can be used as in other embodiments herein; e.g., size, color and the like are applicable here as well. An effect icon may show the trend as well, namely the effect decreasing or increasing at the depicted time, e.g., with an arrow pointing upward or downward.

Fig. 2b further shows medical intervention 241 and 242. Showing the interventions themselves is optional, but may be beneficial to convey a more informative picture of a patient status. Typically, the physiological parameters are shown up to the most recently known values. Typically, the physiological parameters are known and displayed in real-time. Neither is necessary though. For example, visualization 2b may also be used to study previous interventions, e.g., for training or evaluation purposes.

A difference with Fig. 2a is that multiple values of the same physiological parameters are shown in Fig. 2b, whereas in Fig. 2a typically only the current values are visualized. A medical professional may be assisted in making clinical decision using either visualization. Here, Fig. 2b has the advantage of showing a trend of the values.

Fig. 2c also shows a visualization of the history of two physiological parameters against time. Like Fig. 2a, an optional timeline 233, optional visualized interventions 241 and 242 and the effect indicators 251 and 252.

Fig. 2c includes two visualization of a physiological parameter against time: visualizations 261 and 262. Both are graphs against time, however instead of indicating a value of a physiological parameter as a y-coordinate, the value is indicated as a color or texture. For example, low, normal, and high values could be assigned a different color, so that the professional can see at a glance when the value was low, normal, and high, and also how often the values were such. Fig. 2c shows a visualization of an expected effect of a previous medical intervention. Shown are effects 251 and 252. Similar visualization of such effects can be used as in other embodiments herein; e.g., size, color and the like are applicable here as well.

In either Fig. 2a and 2b, an input element may be included, such as a knob or slider, which can be used to shift time further into the past or to a later time. The visualization of the medication effect may then adapt to the selected time. Also here the system may select an interesting time point to show, such as the time at which the effect of the intervention is assumed to be at a maximum.

### C. Scatter view two physiological parameters and effect

Yet a further example of a visualization representing one or more physiological parameters comprises a plot of at least two physiological parameters or a derived parameter against time, wherein at least two physiological or derived parameters are plotted against each other.

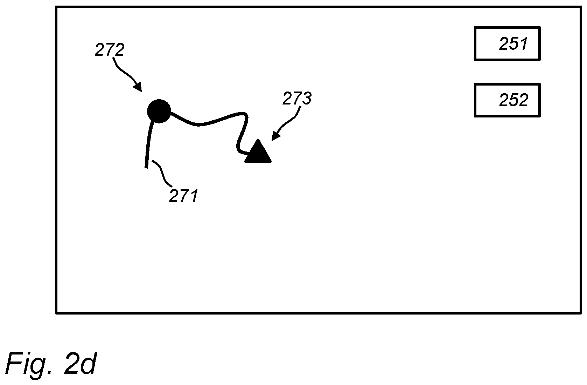

**Fig. 2d** schematically shows an example of an embodiment of a visualization. The two parameters are plotted against each other, e.g., one parameter being indicated with the x-coordinate of a point, while another parameter being indicated with the x-coordinate of a point. If one or both of the parameters change, a curve 271 is plotted. Relevant information may be indicated in the visualization. For example, if a medical intervention took place, this can be indicated; for example, Fig. 2d shows intervention 272. The current time may be indicated with an icon as well; for example, current time is indicated in Fig. 2d with an icon 273. Icon 273 may move along the curve to indicate to the user which point on the curve corresponds to the current time.

Also shown are the effects of previous interventions; shown are effect 251 and effect 252. The expected effect shown in the visualization may be the expected effect at a moment the visualized parameter are measured, e.g., corresponding to the first or last plotted point. The expected effect shown may be an average effect of the visualized time span. The expected effect shown in the visualization may be a current effect, e.g., the expected effect at the moment the visualization is shown to the user.

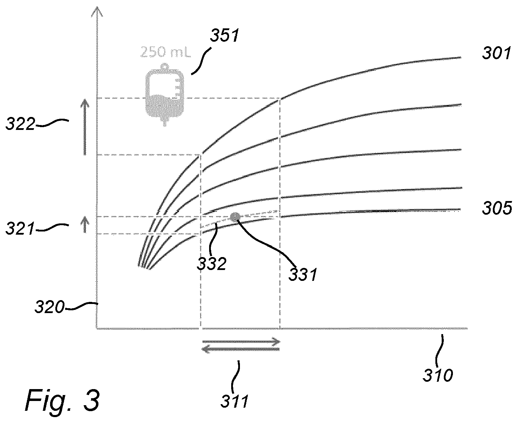

**Fig. 3** schematically shows an example of an embodiment of a visualization.
Shown is a patient visualization in the form of a Frank-Starling curve. This is a plot of the stroke volume vs the left ventricular end-diastolic pressure. Making this plot for a particular patient gives a picture from which the condition of the heart can directly be seen.

Shown in Fig. 3 is a plot of stroke volume versus cardiac preload. Axis 310 indicates cardiac preload. Axis 320 indicates stroke volume. A number of template curves are shown; two of which are indicated with numbers 301 and 305. Curve 301 corresponds to normal ventricular systolic function. Curve 305 shows poor ventricular systolic function.

A fluid challenged was applied to the patient corresponding to the visualization. The fluid challenge is indicated on the cardiac preload axis at 311. On the stroke volume axis two possible responses are indicated: response 321 and 322. Response 321 corresponds to essentially no response to the fluid challenge. Response 322 corresponds to a significant response.

The measured stroke volume and cardiac preload are plotted against each other in curve 322. One point of this curve is indicated at 332.

The visualization also shows an effect visualization 351. Effect visualization 351 indicates the expected remaining effect of the fluid challenge. A medical practitioner can view curve 332 and compare it to the effect amount shown at 351.

The administration of intravenous (IV) fluids is important for the management of critically ill patients. The fluids, typically crystalloid solutions, may be used to resuscitate patients who are hypovolemic or dehydrated, correct free water deficits, replace ongoing fluid losses, and meet the fluid requirements of patients who cannot take fluids enterally. The fluids directly increase the blood volume, without introducing active substances for chemical reactions. The amount of fluid given should be balanced well, because fluid overload may lead to adverse outcomes such as pleural effusion. Some patients, so-called non-responders, poorly respond to fluids, e.g., their cardiac output and stroke volume barely increase by the administration of fluids. This is a contraindication for starting fluid therapy. To find out whether the patient is fluid responsive, usually a fluid challenge is done. In such a fluid challenge, a small bolus of IV fluid is given over a short period of time to assess hemodynamic response.

See Aya HD, et al. "Pharmacodynamic Analysis of a Fluid Challenge" for more information on the dynamic response to a fluid challenge. Like for pharmacokinetics a fluid challenge also has a certain time before the maximum effect is reached. For cardiac output, this time turns out to be 1.2 minute on average (0.6 - 2.8 min, 95% interval) in responders, see Aya. After 10 minutes, there is no evidence of a difference between responders and non-responders for any hemodynamic parameter. Their conclusion is that the change in CO should be assessed one minute after the end of the fluid infusion to see the response compared to just before administration of the fluid.

Fluid therapy thus also has an effect that first becomes noticeable then going to a peak, and later fading out. Also here, after a fluid bolus has been given, effect visualization as described herein could be used to show the intervention becoming effective, the time of maximum effect, and the fading out. For example, the effect visualization may use an icon transparency, saturation, darkness, color, size, animation, arrows, and so on.

Visualization in Fig. 3, shows a Frank-Starling curve plotted from patient's data together with the effect of the fluid challenge. In this case, 250 mL of fluid is administered.

Point 331 gives the stroke volume vs cardiac preload of the patient at or right after (0-10 sec) the time of administration of the fluid for a fluid challenge. As even for responsive patients a fluid challenge would hardly have any effect yet right after administration, the icon representing the fluid may initially be a very light green. In case of retrospectively looking at the patient's data, the dotted curve could be shown as then it is known already what the following values during the fluid challenge will be. On the other hand, in case the data are looked at real-time, there may not be a dotted line.

After 1.2 minutes the maximum effect is expected. The icon 351 may be at its darkest color, e.g., dark green. The dotted line 332 shows the values, either real values or interpolated, from the time of administration till the time of maximum effect. As the curve shows hardly any increase in stroke volume, it can be concluded that this patient is a non-responder. Therefore, he should not get further fluids, as these won't improve his status.

About 5 minutes after the administration of the fluid, the effect is decreasing, which may be represented by icon 351 getting lighter.

### D. medical image + effect

In a further embodiment the visualization representing the physiological parameter comprises a medical image, such as an ultrasound image or ultrasound frames together with the current effect of a previous intervention, e.g., previously applied medication or fluid challenge. The current effect may be the effect at the moment the image is shown. Instead of a current effect, the shown effect may be the effect at the time the image was captured.

Ultrasound is regularly used, e.g., at the ICU, e.g., to assess a patient's cardiac function. Several physiological parameters, such as stroke volume, ejection fraction, cardiac output, left- and right-ventricular volume, can be derived from ultrasound image and/or video. The recordings can be stored as single images or as short videos, e.g., cine loops. Especially when the clinician is looking back to past images/videos, it would be good that he/she has the information about the therapeutic effect of medication and/or fluids with it.

Besides using it to assess the cardiac function, ultrasound can also be used to assess the function of other organs, in particular kidney, for which it may also be important to know the therapeutic effect of medication and/or fluids. Furthermore, ultrasound may be used to look at an embryo or at muscle and/or tendon tissue. Knowledge of a therapeutic effect may be helpful when interpreting such images.

A person skilled in the art will appreciate that the method may be applied to multidimensional image data, e.g., to two-dimensional (2D), three-dimensional (3D) or four-dimensional (4D) images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

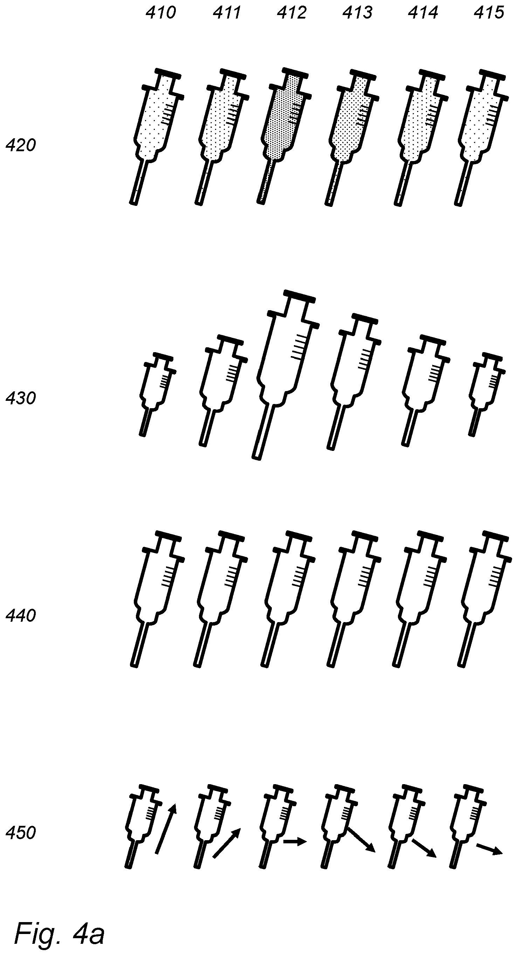

**Fig. 4a** schematically shows examples of an embodiment of a visualization representing an expected effect at a particular point in time. Fig. 4a shows a sequence of icons that may be used to visualize a larger or smaller effect of an intervention, e.g., a medication For example, shown in Fig. 4a, are levels corresponding to, e.g.,
410: right after administration
411: increasing effect
412: maximum effect
413: decreasing effect
414: further decreasing effect
415: almost worn off

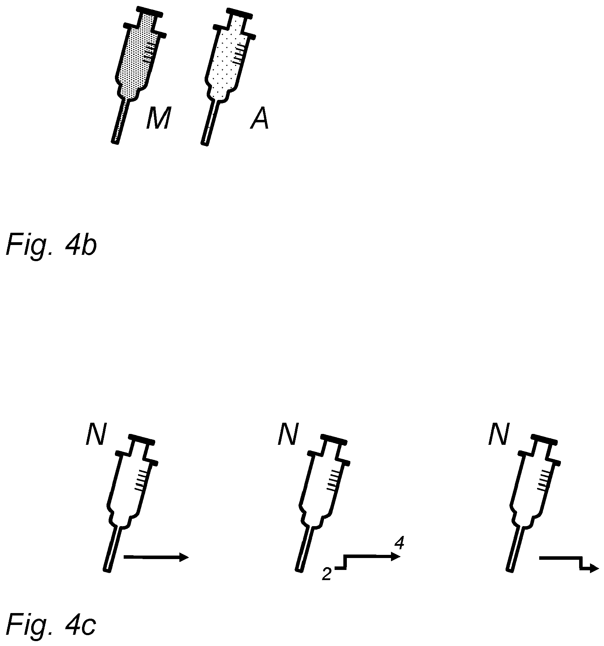

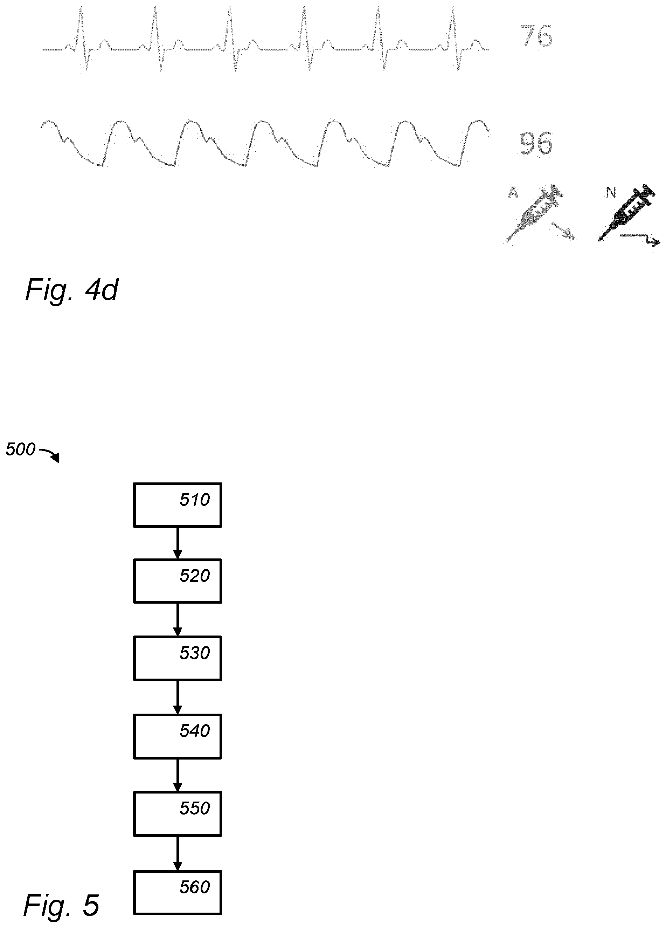

For example, as shown in row 410 effect size is indicated by the density of the fill pattern of the icon. The density increases and decreases as the effect increases and decreases. In Fig. 4 the icon represents a syringe, but other icons may be used.

For example, as shown in row 430 effect size is indicated by the size of the icon. For example, a larger syringe icon may represent a larger effect. For example, in an embodiment, the size of the icon may increase to indicate a build-up of a medication effect, at some point the size of the icon may decrease to indicate a wearing off of the medication effect.

For example, as shown in row 440 effect size is indicated by the darkness of the icon.

Many other visual attributes may be used to visualize an effect size. For example, visual attributes may include transparency, and color scheme, e.g., a rainbow color scheme. For example, an icon may change have the colors: red, orange, yellow, green, blue, and purple to indicate the effect stages 410-415. In an embodiment, a different visualization is used for an increasing part of the effect, e.g., stages 410-412, and for a decreasing part of the effect, e.g., stages 412-415. This has the effect, that even if the effect value is the same, a user can see at a glance if the effect is likely to increase or decrease. For example, even if, say, the effect sizes at 411 and 414 are the same in absolute value, by visualizing them differently, e.g., coloring them different, e.g., using a different point in a color scale, such as a rainbow color scale, a practitioner can immediately see, that at 411 the size is likely to increase, while at 414 it is likely to decrease.

In addition (or instead) of a visualized expected effect, e.g., a visualization of a magnitude of the effect, the visualization may comprise a visualization of effect trend, e.g., whether the effect is increasing or decreasing. For example in row 450 an arrow indicates the effect trend. An upward pointing arrow may indicate a rising trend, while a decreasing arrow may indicate a decreasing trend. Different aspects may be visualized. For example, a trend size may be visualized, e.g., by an angle of the arrow. For example, an effect magnitude may be visualized, e.g., by the size of the arrow.

The visualization icon may also indicate multiple dimensions of the effect; for example, the icon may both show the magnitude of the current effect, and its trend, e.g., whether the effect is currently increasing, decreasing or stable.

Multiple dimensions may be indicated by combining multiple visual styles, e.g., color and darkness for two respective dimensions. Multiple dimensions may be indicated by combining a visual style with an orientation of the icon. For example, to show an increasing trend a syringe may be shown point-upward, while for a decreasing trend the syringe may be shown point-downwards. A second dimension may be indicated with a further icon, e.g., an arrow, a letter, or the like.

The icon may be or comprise one or more numbers and/or letters, e.g., instead of a drawing. For example, 10 may mean maximum effect, e.g., a number that can range between 0 and 10. A visual attributed of the letter and/or number, e.g., its color, may indicate the type of medication. For example, a letter may refer to the type of medication, which, when colored red, means that the medication has reached its maximum effect.

**Fig. 4b** schematically shows examples of an embodiment of a visualization representing an expected effect at a particular point in time. In this example, the icons indicate the medical intervention to which they relate. In this case, a capital letter is added to indicate commonly used medication. As an example, medication 'M' (e.g., Milrinone) may have been taken some time ago and its concentration in blood is declining, whereas medication 'A' (e.g., noradrenaline) is being administered at a rate which has recently been lowered, but there is still an effect of the higher rate it had before.

Other types of medication could be indicated with another letter or letter combination (e.g., 'Do' for dobutamine, or just the full name 'dobutamine') and/or another color. For oral medication, the icon might be adapted to pills or a drink. A number might be added to the icon to reflect the amount (e.g., '2' when two pills are taken or '4' when 4 milliliter is given). A fluid for fluid management might be shown as a fluid bag. Oxygen therapy might be shown as 'O2', mechanical ventilation by a ventilator image. Drinks or food might also be depicted; especially for neonate monitoring, it might be important to know when they drink.

Effects of multiple types of medications/interventions can take place at the same time. The visualization can thus contain multiple icons, each depicting a type of medication/intervention. The type of medication/intervention might be depicted with a letter (e.g., 'M' for Milrinone and 'A' for another type of medication, e.g., a type of medication starting with letter 'A'), type of icon (e.g., instead of a syringe, pills might be shown for a type of medication with oral intake), or with the color of the icon (e.g., Milrinone is depicted with a purple syringe, while a particular other type of medication is shown with a green syringe). Preferably, the type of medication and medication effect are not both indicated with a color to avoid confusion.

Each intervention, e.g., type of medication, might have a fixed position on the screen, as soon as it becomes applicable, always lights up at the same place on the dashboard. Alternatively, the order of icons shown depends on the order it has been given (e.g., the one in the upper left corner is always the one that has been applied latest), or on the current effect (e.g., the one in the upper left corner is always the one that currently has the largest effect).

A color scheme or an additional arrow has an advantage over the other ways of showing the medication effect (transparency, darkness, and size). Namely, with the color scheme or with the arrow, it can be seen from a single screenshot, where on the curve the patient would be; e.g., it can be distinguished if the medication concentration in the blood is increasing or decreasing.

Cumulative effects of administrating a new bolus before the previous one has fully worn off may also be taken into account. This could for example be done by, right after admission of the second bolus, directly go to a darker icon than it was before instead of starting all over with a light icon.

Some types of medication are administered continuously (e.g., via an infusion pump). This could be depicted with a horizontal arrow, as the effect will not change in time, as long as the given rate is constant and has been given for some time already. Here transparency, darkness, color, or size could represent the rate of the medication given, or a number might be added to give this information, e.g., 4 to represent that the medication is given at a rate of 4 milliliter per hour. Here also an arrow could be used to represent how long the concentration has already been on the given number and/or where it is coming from. When it has already been on a certain concentration for so long that the blood concentration won't fluctuate anymore, this could be represented by a long horizontal arrow. When the amount has been increased or decreased recently, e.g., within the past 15 minutes, this might be shown as a step-like arrow.

**Fig. 4c** schematically shows examples of an embodiment of a visualization representing an expected effect at a particular point in time. Fig. 4c shows the continuous administration of medicine, indicated by a letter N in the visualization, e.g., noradrenaline. For example, the administration may be via infusion pump. In the left visualization, the rate has been constant for a long time. In the middle visualization, the rate changed some time ago from 2 milliliter per hour to 4 milliliter per hour. There might still be a small influence of the previous rate on the blood concentration, which is indicated by a small horizontal part lower on the left-hand side of the arrow. In the right visualization, the rate changed recently from a higher amount to a lower amount. An influence of the higher amount on the blood concentration is indicated by a long horizontal part higher on the left-hand side of the arrow.

**Fig. 4d** schematically shows examples of an embodiment of a visualization representing an expected effect at a particular point in time. Fig. 4d schematically shows how different elements may be combined in a visualization. For example, Fig. 4d may be shown on a patient monitor. For example, a conventional patient monitor may be adapted according to an embodiment.

Shown in Fig. 4d are semi-current traces, e.g., showing a time period covering the past minute, or past 30 seconds, or less, of a physical parameters, e.g., sensor values. In this example, two parameters are plotted, but more or fewer parameters could be shown. For example, Fig. 4d may be shown on a display of a patient monitor.

In Fig. 4d, the upper trace represents an ECG-waveform and the number next to it (76) represents the current heart rate. The lower trace represents a PPG-waveform, e.g., of an SpO2-sensor and the number next to it (96) represents the oxygen saturation (SpO2). In the right bottom corner indicators show the therapeutic effects of the two types of medication indicated with the letters A and N.

Two intervention effects are shown. For medicine A, the effect is currently high, but the trend is decreasing. Medicine N may be an intravenous medicine, e.g., as shown in Fig. 4c. For example, the icon may show that medicine N is still how but expected to decrease due to a changed configuration. In this case, the future expected effect is shown.

**Fig. 5** schematically shows an example of an embodiment of a visualization method 500. Visualization method (500) is configured for showing a patient status. The visualization method comprises:
- receiving (510) physiological parameters indicative of a patient status,
- showing (520) a visualization of the patient status,
- obtaining (530) a medical intervention that has been applied to the patient at a first point in time,
- computing (540) an effect that the medical intervention is expected to have on the patient at a second point in time,
- generating (550) a visualization representing the physiological parameter at the first and/or second time point, and the expected effect at the second time point,
- causing (560) the generated visualization to be displayed on the display through the display interface.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 500. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

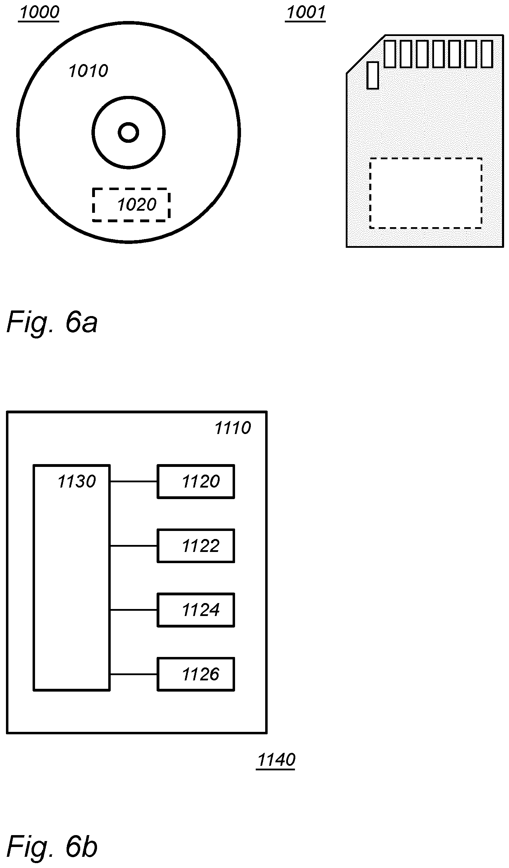

**Fig. 6a** shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform a visualization method according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said visualization method.

**Fig. 6b** shows in a schematic representation of a processor system 1140 according to an embodiment of a visualization device. The processor system comprises one or more integrated circuits 1110. The architecture of the one or more integrated circuits 1110 is schematically shown in Fig. 6b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the visualization device may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While device 1140 is shown as including one of each described component, the various components may be duplicated in various embodiments. For example, the processor may include multiple microprocessors that are configured to independently execute the methods described herein or are configured to perform steps or subroutines of the methods described herein such that the multiple processors cooperate to achieve the functionality described herein. Further, where the device 1140 is implemented in a cloud computing system, the various hardware components may belong to separate physical systems. For example, the processor may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A visualization device for showing a patient status, the visualization device comprising:
- a communication interface configured to receive physiological parameters indicative of a patient status,
- a display interface configured to enable a display to show a visualization of the patient status,
- a processor system configured to
- obtain a medical intervention that has been applied to the patient at a first point in time,
- compute an effect that the medical intervention is expected to have on the patient at a second point in time,
- generate a visualization representing the physiological parameter at the first and/or second time point, and the expected effect at the second time point,
- causing the generated visualization to be displayed on the display through the display interface.

2. A visualization device as in Claim 1, wherein the physiological parameters are shown in real time.

3. A visualization device as in any one of the preceding claims, wherein the second time point corresponds to the moment when the generated visualization is displayed.

4. A visualization device as in any one of the preceding claims, wherein the generated visualization represents a snapshot of the physiological parameter at the second time point.

5. A visualization device as in any one of the preceding claims, wherein the visualized expected effect comprises a visualization of magnitude and/or trend of the effect.

6. A visualization device as in any one of the preceding claims, wherein the visualization representing the physiological parameter comprises one or more of:
- a trend view of the physiological parameter or a derived parameter against time, wherein the parameters is plotted against time,
- a scatter view of the physiological parameters or a derived parameter, wherein at least two physiological or derived parameters are plotted against each other,
- a graphical model of at least part of the patient indicating one or more physiological parameters, such as a cardiopulmonary (CP) model or visual representation of the patient, and/or
- a medical image, e.g., an ultrasound image or ultrasound frame(s).

7. A visualization device as in any one of the preceding claims, wherein the intervention comprises administration of a medically active composition, the effect comprising a computed plasma concentration in the patient.

8. A visualization device as in any one of the preceding claims, wherein the physiological parameters comprise
- one or more vital signs, including one or more of: heart rate (HR), electrocardiography (ECG), respiration rate (RR), core body temperature (CBT), oxygen saturation (SpO2) and blood pressure (BP), and/or
- one or more hemodynamic parameters, including one or more of: cardiac output (CO), stroke volume (SV), stroke volume variation (SVV), ejection fraction (EF), cardiac index (CI), systemic vascular resistance (SVR), pulmonary vascular resistance (PVR), pulmonary artery pressure (PAP), and extravascular lung water index (ELWI),
- one or more of blood parameters, including blood gas, e.g., blood pH, blood COOH, and/or blood values, e.g., blood Hb, blood Ht, blood Hct, and/or
- urine output.

9. A visualization device as in any one of the preceding claims, wherein the interventions are obtained from:
- an electronic medical record (EMR),
- an electronic medical device, e.g., an infusion pump, or a ventilator.

10. A visualization device as in any one of the preceding claims, wherein the first time point is estimated from a measured effect of the intervention.

11. A visualization device as in any one of the preceding claims, wherein the processor system is configured to obtain multiple medical interventions, the effects of which overlap in time.

12. A visualization device as in any one of the preceding claims, wherein the medical intervention is a fluid challenge, the computed effect indicating a time from maximum effect of the fluid challenge on the patient.

13. A visualization device as in Claim 12, wherein the generated visualization comprises a plot of the stroke volume vs a diastolic pressure.

14. A visualization method (500) showing a patient status, the visualization method comprising:
- receiving (510) physiological parameters indicative of a patient status,
- showing (520) a visualization of the patient status,
- obtaining (530) a medical intervention that has been applied to the patient at a first point in time,
- computing (540) an effect that the medical intervention is expected to have on the patient at a second point in time,
- generating (550) a visualization representing the physiological parameter at the first and/or second time point, and the expected effect at the second time point,
- causing (560) the generated visualization to be displayed on the display through the display interface.

15. A transitory or non-transitory computer readable medium (1000) comprising data (1020) representing instructions, which when executed by a processor system, cause the processor system to perform the method according to Claim 14.
